Europäisches Patentamt

European Patent Office

Office européen des brevets

(11) Publication number: **0 161 481**
**B1**

(12) **EUROPEAN PATENT SPECIFICATION**

(45) Date of publication of patent specification: **12.09.90**

(51) Int. Cl.[5]: **C 12 Q 1/04**

(21) Application number: **85104307.5**

(22) Date of filing: **10.04.85**

(54) Medium for citrate utilization test.

(30) Priority: **09.05.84 JP 91067/84**

(43) Date of publication of application:
**21.11.85 Bulletin 85/47**

(45) Publication of the grant of the patent:
**12.09.90 Bulletin 90/37**

(84) Designated Contracting States:
**BE DE FR GB IT**

(56) References cited:
**EP-A-0 059 113**
**GB-A-1 554 193**
**US-A-4 048 016**
**US-A-4 335 205**

**PATENT ABSTRACTS OF JAPAN, vol. 6, no. 3
(C-86)881r, 9th January 1982; & JP-A-56 127 099
(NIPPON TECTRON K.K.) 05-10-1981**

(73) Proprietor: **TERUMO KABUSHIKI KAISHA
trading as TERUMO CORPORATION
44-1, 2-chome, Hatagaya Shibuya-Ku
Tokyo 151 (JP)**

(72) Inventor: **Igarashi, Takeshi
31-13, 1-chome, Suwa
Tama-shi Tokyo (JP)**
Inventor: **Endo, Toshihiko
1417-1, Miyajima
Fuji-shi Shizuoka-ken (JP)**
Inventor: **Koshi, Isei
3350-1, Mannohara-shinden
Fujinomiya-shi Shizuoka-ken (JP)**

(74) Representative: **Henkel, Feiler, Hänzel & Partner
Möhlstrasse 37
D-8000 München 80 (DE)**

Courier Press, Leamington Spa, England.

# EP 0 161 481 B1

**Description**

## BACKGROUND OF THE INVENTION
### Field of the Invention

This invention relates to a medium for testing microorganisms for biochemical behavior. More particularly, this invention relates to an improved medium to be used in performing citrate utilization test on microorganisms such as an enteric bacteria by the method of biochemical identification.

For administration of a proper medication to a patient of an infectious disease, it is essential to identify a pathogenic microorganism responsible for the disease, subjecting the patient to a sensitivity test, and selecting an effective medicine. In the identification of such a pathogenic microorganism, many biochemical tests are performed. One of them is a citrate utilization test. The medium of this invention is advantageously used for the citrate utilization test.

### Description of the Prior Art

The citrate utilization test is intended to determine whether or not citric acid can be utilized as a carbon source in the culture of a given microorganism. At present, it is one of the most important tests performed on microorganisms for biochemical behavior. This test is performed by culturing the given microorganism in a medium containing citrate and a pH indicator as main components. Recently, as a medium for use in this test, a composition comprising yeast extract, sodium citrate, glucose, glutathione (reduction type), nicotine adenine dinucleotide, sodium chloride, disodium monohydrogen phosphate, bromothymol blue and purified water has been proposed (Japanese Patent Publication No. SHO 58(1983)—20263). This medium permits the time required for culture to be notably decreased as compared with the conventional counter type. Specifically, the culture time has been one to two days in the conventional medium, whereas it is four to five hours in the medium under discussion, making it possible to conduct the test and find the result of the test on one same day. Depending on the kind of the microorganism under test, however, the culture performed for four to five hours in this medium does not permit the reaction to proceed sufficiently for required evaluation. In this case, the culture time must be elongated and the evaluation of the test result made on the following day. In the circumstance, the desirability of developing a medium in which any microorganism can be cultured sufficiently in a matter of four to five hours to permit clear distinction between positive and negative test of the reaction has found growing approval.

For early medication, the identification of a pathogenic microorganism is desired to be carried out as rapidly as permissible. There are, however, times when the evaluation of the test result is completed to be performed on the following day because of the convenience of the work involved in the test. In this case, the medium is desired to be such that the culture time is not rigidly specified and, therefore, the culture time may be elongated to suit the convenience of the work and, despite the elongation of the culture time, the reaction is not suffered to proceed excessively and the evaluation of the test result can be conducted accurately.

For the efficiency of work involved, many biochemical tests are frequently performed all at once by the use of a multi-well culture plate. Thus, it becomes necessary for culture times of different test items to be equalized to one another. Again in this case, the medium is desired not to involve any rigid limitation of culture time.

An object of this invention, therefore is to provide a novel medium for the citrate utilization test.

Another object of this invention is to provide a medium for the citrate utilization test which does not specifically limit culture time and enables the identification of the pathogenic microorganism to be performed on the same day as the culture is carried out or on the day following the culture.

A further object of this invention is to provide a medium for citrate utilization test which permits a color reaction to produce a distinct color and enables the evaluation of test result to be effected with ease.

### Summary of the Invention

The objects described above are attained by a medium for the citrate utilization test, which comprises 0.5 to 5 g of a citrate, 0.1 to 1 g of dialkali metal monohydrogen phosphate, 0.2 to 2 g of monoalkali metal dihydrogen phosphate, 0.3 to 6 g of magnesium salt, 1 to 10 g of sodium chloride and 0.01 to 0.2 g of a pH indicator, wherein the component ratio of dialkali metal monohydrogen phosphate to monoalkali metal dihydrogen phosphate by weight is in the range of 1:1.5 to 1:5, which medium assumes a pH value in the range of 5.5 to 7.0 when dissolved in 1 liter of water.

### Description of the Preferred Embodiment

The medium of this invention comprises citrate, dialkali metal monohydrogen phosphate, monoalkali metal dihydrogen phosphate, magnesium salt, sodium chloride and pH indicator.

The composition of this medium comprises 0.5 to 5 g, preferably 1 to 4 g of citrate, 0.1 to 1 g, preferably 0.2 to 0.6 g of dialkali metal monohydrogen phosphate, 0.2 to 2 g, preferably 0.3 to 1.2 g of monoalkali metal dihydrogen phosphate, 0.3 to 6 g, preferably 0.5 to 3 g of magnesium salt, 1 to 10 g, preferably 2 to 8 g of sodium chloride and 0.01 to 0.2 g, preferably 0.06 to 0.14 g of a pH indicator in 1 liter of water, wherein the component ratio of dialkali metal monohydrogen phosphate to monoalkali metal dihydrogen phosphate by weight is in the range of 1:1.5 to 1:5, preferably 1:2 to 1:3.

2

The medium in accordance with the present invention has a feature that dialkali metal monohydrogen phosphate and monoalkali metal dihydrogen phosphate as pH adjusters control buffering ability of the medium. If the buffering ability is suppressed, it is liable to be pseudo-positive when the determination is carried out next day, because it is designed that the reaction occurs in a short time cultivation. If the buffering activity is hastened in order to avoid such defect, it becomes difficult to occur the reaction in the short time. Thus it is necessary to use specified amounts of dialkali metal monohydrogen phosphate and monoalkali metal dihydrogen phosphate, namely the amount in which the component ratio of dialkali metal monohydrogen phosphate to monoalkali metal dihydrogen phosphate by weight is in the range of 1:1.5 to 1:5, in order to make possible either on the same day and the following day. As the alkali metal salts thereof, potassium or sodium salts are preferable.

As for magnesium salt, a mineral acid salt, such as magnesium sulfate, magnesium chloride, magnesium nitrate or magnesium phosphate, is preferably usable.

As for citrate, an alkali metal, such as sodium, potassium or lithium, or ammonium salt of citric acid is usable.

Although this invention does not discriminate the pH indicator by its kind, the pH indicator is desired to be phenol red, bromothymol blue, bromocresol purple, cresol red, bromophenol red, chlorophenol red and the like. Bromothymol blue is best choice. The amount of the pH indicator is to be varied to some extent with its kind. For example, as bromothymol blue is used, the amount thereof is desired to be in the range of 0.06 to 0.14 g per a liter of water.

The proportions of the components in the medium of this invention are critical. In particular, the proportion of the monoalkali metal dihydrogen phosphate and the dialkali metal monohydrogen phosphate is fixed so that the pH value of the medium will fall in the range of 5.5 to 7.0, preferably 6.0 to 6.5, and the color of the color reaction will appear distinctly. Also the proportion of the citrate is selected so as to permit reduction in the culture time.

Further, for the medium of the present invention, the proportion of the pH indicator and the proportion of the magnesium salt are selected in order that the color of the color reaction is distinct enough to permit easy discrimination between positive and negative test even when the amount of the sample size is small.

The medium of this invention is prepared for use by dissolving the components in their respective proportions in water. In recent years, it is normal for numerous tests to be performed all at once by the use of a multi-well culture plate. For the medium of this invention to be used in this manner, it is poured into the wells of the culture plate and dried to be used as dry medium. In the test, a suspension of a microorganism is dispensed in a prescribed volume to the individual wells and left standing therein to effect required culture for a prescribed length of time. By the change in color of the culture solution, the discrimination between positive test and negative test of the citrate utilization test is effected.

The medium of the present invention is used in much the same way as the conventional medium for citrate utilization test. With the medium of this invention, although the culture time can be reduced to the order of three to five hours, it is not specifically limited to that range. The culture may be performed for a longer period. Thus, the culture medium permits the evaluation of the test result either on the same day as the sample is taken or on the following day. The color produced in color test is so distinct as to permit easy discrimination between positive test and negative test of the reaction.

Now, one working example of the actual use of the medium of this invention in the citrate utilization test will be described below.

Examples I-VI and Controls I-III

| composition of medium | Example | | | | | | Control | | |
|---|---|---|---|---|---|---|---|---|---|
| | I | II | III | IV | V | VI | I | II | III |
| Yeast extract (g)* | – | – | – | – | – | – | 1.0 | – | – |
| Glutathione, reduced form (g) | – | – | – | – | – | – | 3.0 | – | – |
| NAD (g)** | – | – | – | – | – | – | 0.05 | – | – |
| Disodium monohydrogen phosphate (g) | – | – | – | – | – | – | 0.55 | – | – |
| Dipotassium monohydrogen phosphate (g) | 0.35 | 0.35 | 0.1 | 0.6 | 0.2 | 0.2 | – | 0.35 | 1.5 |
| Monopotassium dihydrogen phosphate (g) | 0.7 | 0.7 | 0.4 | 1.2 | 0.3 | 0.6 | – | 0.7 | 1.5 |
| Magnesium sulfate (g) | 1.0 | 1.0 | 1.0 | 3.0 | 0.5 | 0.5 | – | 0.2 | 1.0 |
| Sodium chloride (g) | 5.0 | 5.0 | 5.0 | 8.0 | 2.0 | 2.5 | 4.0 | 5.0 | 5.0 |
| Trisodium citrate (g) | 2.0 | 2.0 | 2.0 | 4.0 | 1.0 | 1.0 | 6.0 | 2.0 | 2.0 |
| Bromothymol blue (g) | 0.12 | 0.08 | 0.12 | 0.14 | 0.06 | 0.1 | 0.14 | 0.12 | 0.12 |
| Purified water (liter) | 1 | 1 | 1 | 1 | 1 | 1 | 1 | 1 | 1 |
| pH | 6.4 | 6.4 | 6.2 | 6.0 | 6.5 | 6.4 | 6.0 | 6.4 | 6.4 |

\* Yeast extract produced by Oxoid Corp.

\*\* NAD: nicotinamide adenine dinucleotide

EP 0 161 481 B1

Culture conditions

The medium of this invention and control media having the compositions as shown above were dispensed in a unit volume of 50 µl to the wells of a culture plate and dried at 40°C. Separately various microorganisms were cultured on agar culture plates at 35° to 37°C for 18 to 24 hours and suspended in 1.0 ml sterilized distilled water in a concentration of about 6 to 9 × 10⁸ cells/ml. The suspensions were inoculated in a unit volume of 50 µl to the dry medium and, under a cover, cultured at 35° to 37°C for a prescribed length of time. By the change in color of the resultant solutions, presence or absence of the citric acid decomposition reaction was evaluated. The results are shown in Table 1.

Table 1

| Micororganism | Medium of Examples I-VI | | Control medium I | | Control medium II | | Control medium III | |
|---|---|---|---|---|---|---|---|---|
| | 4 to 5 hours' culture | 16 to 20 hours' culture | 4 to 5 hours' culture | 16 to 20 hours' culture | 4 to 5 hours' culture | 16 to 20 hours' culture | 4 to 5 hours' culture | 16 to 20 hours' culture |
| *Klebsiella oxytoca* | + | + | + | + | + | + | + | + |
| *Escherichia coli* | - | - | - | - | - | - | - | - |
| *Morganella morganii* | - | - | - | - | - | - | - | - |
| *Proteus mirabilis* | + | + | - | + | ± - + | + | ± - - | ± - + |
| *Providencia rettgeri* | + | + | - | + | ± - + | + | ± - - | ± - + |
| *Enterobacter sakazakii* | + | + | - | + | + | + | ± - + | + |
| *Klebsiella pneumoniae* | + | + | - | + | ± - + | + | ± - - | ± - + |
| *Serratia marcescens* | + | + | - | + | ± - - | + | - | ± - + |
| *Serratia marinorubra* | + | + | + | + | + | + | ± - + | + |
| *Serratia liquefaciens* | + | + | ± - + | + | ± - + | + | ± - - | ± - + |

+ : Color of positive test
+ - + : Intermediate color closer to color of positive test
± - - : Intermediate color closer to color of negative test
- : Color of negative test

EP 0 161 481 B1

# EP 0 161 481 B1

It is noted from the results of Table 1 that the use of the medium of this invention in the citrate utilization test permits accurate identification of microorganisms without reference to length of culture time. Thus, with the medium of this invention, the evaluation of test result can be carried out either on the same day as the sample is taken or on the following day, depending on the convenience of the work involved in the test.

In contrast, in the use of control media, the color produced after 4 to 5 hours' culture is not amply distinct to permit the evaluation of test result on the same day as the sample is taken.

Moreover, the fact that the medium of this invention is not rigidly restricted by the length of culture time proves highly convenient where numerous biochemical tests are carried out all at once.

**Claims**

1. A medium for citrate utilization test, comprising:
0.5 to 5 g of citrate,
0.1 to 1 g of dialkali metal monohydrogen phosphate,
0.2 to 2 g of monoalkali metal dihydrogen phosphate,
0.3 to 6 g of magnesium salt,
1 to 10 g of sodium chloride, and
0.01 to 0.2 g of a pH indicator,
wherein the component ratio of dialkali metal monohydrogen phosphate to monoalkali metal dihydrogen phosphate by weight is in the range of 1:1.5 to 1:5, which culture medium exhibits a pH value in the range of 5.5 to 7.0 when dissolved in 1 liter of water.

2. A medium according to Claim 1, which comprises:
1 to 4 g of citrate,
0.2 to 0.6 g of dialkali metal monohydrogen phosphate,
0.3 to 1.2 g of monoalkali metal dihydrogen phosphate,
0.5 to 3 g of magnesium salt,
2 to 8 g of sodium chloride,
0.06 to 0.14 g of a pH indicator,
wherein the component ratio of dialkali metal monohydrogen phosphate to monoalkali metal dihydrogen phosphate by weight is in the range of 1:2—1:3.

3. A medium according to Claim 2, wherein the pH value of the medium is in the range of 6.0 to 6.5.

4. A medium according to Claim 1, wherein said citrate is an alkali metal or ammonium salt.

5. A medium according to Claim 4, wherein said alkali metal citrate is potassium or sodium citrate.

6. A medium according to Claim 1, wherein said dialkali metal monohydrogen phosphate and monoalkali metal dihydrogen phosphate are potassium or sodium salts.

7. A medium according to Claim 1, wherein said magnesium salt is a mineral acid salt.

8. A medium according to Claim 7, wherein said mineral acid is one compound selected from the group consisting of chloride, nitrate, sulfate and phosphate.

9. A medium according to Claim 1, wherein said pH indicator is one compound selected from the group consisting of phenol red, bromothymol blue, bromocresol purple, cresol red and bromophenol red.

10. A medium according to Claim 9, wherein said pH indicator is bromothymol blue.

**Patentansprüche**

1. Medium für einen Citratausnutzungstest, enthaltend
0,5—5 g Citrat;
0,1—1 g eines Dialkalimetallmonohydrogenphosphats;
0,2—2 g eines Monoalkalimetalldihydrogenphosphats;
0,3—6 g eines Magnesiumsalzes;
1—10 g Natriumchlorid und
0,01—0,2 g eines pH-Indikators,
wobei das Gewichtsverhältnis Dialkalimetallmonohydrogenphosphat/Monoalkalimetalldihydrogenphosphat im Bereich von 1:1,5 bis 1:5 liegt und das Kulturmedium — nach dem Auflösen in 1 l Wasser — einen pH-Wert im Bereich von 5,5—7,0 aufweist.

2. Medium nach Anspruch 1, enthaltend
1—4 g Citrat;
0,2—0,6 g Dialkalimetallmonohydrogenphosphat;
0,3—1,2 g Monoalkalimetalldihydrogenphosphat;
0,5—3 g eines Magnesiumsalzes;
2—8 g Natriumchlorid und
0,06—0,14 g eines pH-Indikators,
wobei das Gewichtsverhältnis Dialkalimetallmonohydrogenphosphat/Monoalkalimetalldihydrogenphosphat im Bereich von 1:2 bis 1:3 liegt.

3. Medium nach Anspruch 2, dadurch gekennzeichnet, daß der pH-Wert des (Kultur-)Mediums im Bereich von 6,0—6,5 liegt.

7

4. Medium nach Anspruch 1, dadurch gekennzeichnet, daß das Citrat aus einem Alkalimetall- oder Ammoniumsalz besteht.

5. Medium nach Anspruch 4, dadurch gekennzeichnet, daß das Alkalimetallcitrat aus Kalium- oder Natriumcitrat besteht.

6. Medium nach Anspruch 1, dadurch gekennzeichnet, daß das Dialkalimetallmonohydrogenphosphat und Monoalkalimetalldihydrogenphosphat aus den Kalium- oder Natriumsalzen bestehen.

7. Medium nach Anspruch 1, dadurch gekennzeichnet, daß das Magnesiumsalz aus einem Mineralsäuresalz besteht.

8. Medium nach Anspruch 7, dadurch gekennzeichnet, daß das Mineralsäuresalz aus Chlorid, Nitrat, Sulfat und Phosphat ausgewählt ist.

9. Medium nach Anspruch 1, dadurch gekennzeichnet, daß der pH-Indikator aus der Gruppe Phenolrot, Bromthymolblau, Bromcresolpurpur, Cresolrot und Bromphenolrot ausgewählt ist.

10. Medium nach Anspruch 9, dadurch gekennzeichnet, daß der pH-Indikator aus Bromthymolblau besteht.

## Revendications

1. Milieu pour le test d'utilisation du citrate, comprenant:
0,5 à 5 g de citrate,
0,1 à 1 g d'un monohydrogénophosphate dialcalin,
0,2 à 2 g d'un dihydrogénophosphate monoalcalin,
0,3 à 6 g d'un sel de magnésium,
1 à 10 g de chlorure de sodium, et
0,01 à 0,2 g d'un indicateur de pH,
dans lequel le rapport pondéral des composants entre le monohydrogénophosphate dialcalin et le dihydrogénophosphate monoalcalin est compris dans la gamme de 1:1,5 à 1:5, lequel milieu de culture présente un pH compris dans la gamme de 5,5 à 7,0 lorsqu'il est dissous dans 1 litre d'eau.

2. Milieu selon la revendication 1, qui comprend:
1 à 4 g de citrate,
0,2 à 0,6 g d'un monohydrogénophosphate dialcalin,
0,3 à 1,2 g d'un dihydrogénophosphate monoalcalin,
0,5 à 3 g d'un sel de magnésium,
2 à 8 g de chlorure de sodium,
0,06 à 0,14 g d'un indicateur de pH,
dans lequel le rapport pondéral des composants entre le monohydrogénophosphate dialcalin et le dihydrogénophosphate monoalcalin est compris dans la gamme de 1:2 à 1:3.

3. Milieu selon la revendication 2, dans lequel le pH du milieu est compris dans la gamme de 6,0 à 6,5.

4. Milieu selon la revendication 1, dans lequel ledit citrate est un sel de métal alcalin ou d'ammonium.

5. Milieu selon la revendication 4, dans lequel ledit citrate de métal alcalin est le citrate de potassium ou de sodium.

6. Milieu selon la revendication 1, dans lequel ledit monohydrogénophosphate dialcalin et ledit dihydrogénophosphate monoalcalin sont des sels de potassium ou de sodium.

7. Milieu selon la revendication 1, dans lequel ledit sel de magnésium est un sel d'acide minéral.

8. Milieu selon la revendication 7, dans lequel ledit acide minéral est un composé choisi dans le groupe formé par un chlorure, un nitrate, un sulfate et un phosphate.

9. Milieu selon la revendication 1, dans lequel ledit indicateur de pH est un composé choisi dans le groupe formé par le rouge de phénol, le bleu de bromothymol, le violet de bromocrésol, le rouge de crésol et le rouge de bromophénol.

10. Milieu selon la revendication 9, dans lequel ledit indicateur de pH est le bleu de bromothymol.